# EUROPEAN PATENT SPECIFICATION

(11) **EP 2 252 241 B1**
(45) Date of publication and mention of the grant of the patent: **11.09.2013**
(21) Application number: 09702934.2
(22) Date of filing: 17.01.2009
(51) Int. Cl.: A61F 5/01

(54) **ANKLE-FOOT ORTHOSES**
FUSSGELENK-FUSS-ORTHESE
ORTHÈSES CHEVILLE-PIED

(30) Priority: 17.01.2008 GB 0800833
(43) Date of publication of application: 24.11.2010
(73) Proprietor: Watts, Robert John, Fordingbridge Hampshire SP6 2PJ (GB)
(72) Inventor: Watts, Robert John, Fordingbridge Hampshire SP6 2PJ (GB)
(74) Representative: Milhench, Mark Lorne
(86) International application number: PCT/EP2009/050522
(87) International publication number: WO 2009/090259

(56) References cited:
- EP-A- 0 619 102
- WO-A-87/02885
- WO-A-99/34757
- GB-A- 2 330 309
- GB-A- 2 420 716
- US-A- 5 217 431
- US-A1- 2003 233 062
- US-A1- 2004 082 895
- US-B1- 7 101 346

## Description

### Field of the Invention

This invention relates to a resilient flexible structure and a method of fitting a resiliently flexible structure to the foot and lower leg of a patient, and yet another embodiment of the invention relates to a method of manufacturing a resiliently flexible structure for fitting to the foot and lower leg of a patient in accordance with the aforementioned fitting method.

### Background to the Invention

Orthoses are mechanical devices which impose forces upon a limb of a patient and which can be used for a variety of different purposes. For example, orthoses can be provided for supportive, functional, corrective or protective purposes, or indeed for a combination of these.

Ankle-foot orthoses are typically used to provide protection to the ankle and foot of a patient as well as to provide support against excessive plantarflexion, or "foot-drop" as it is more colloquially known. Foot plantarflexion is a medical condition that results from disease, trauma or congenital abnormality. Patients affected by the condition typically experience difficulty in walking as their feet tend to drop when lifted off the ground, and to avoid stumbling they typically have to lift their foot higher than they would otherwise have to. It is also not atypical for patients to have problems during the swing-through phase of their gait cycle, as a typical sufferer will tend also to exhibit poor, or impaired, dorsiflexion.

The primary function of an ankle-foot orthosis is to provide a resistance to plantarflexion which helps keep the patient's foot in the correct position when the foot is lifted off the ground. As well as this resistive function, a good ankle-foot orthosis should also provide a degree of assistance to dorsiflexion during the swing-through phase of the patient's gait.

A variety of different ankle-foot orthoses have previously been proposed for resisting plantarflexion, and in some cases for additionally assisting dorsiflexion.

One previously proposed device is commonly known in the art as an "under foot" orthosis. As this colloquial name suggests, the orthosis fits under the foot, and in this case outside of a shoe. This particular device cannot be worn without a shoe, and as such the shoe is an integral component of the orthosis. The orthosis comprises a pair of supporting metal rods, one connected to either side of the shoe in the region of the heel by means of a plantarflexion stop that prevents further foot drop. The upper ends of the rods are connected to a supporting band which is secured about the calf of a patient.

Another previously proposed "under foot" orthosis (which must also be used with a shoe) comprises a rigid one-piece plastics moulding composed of integral sole-abutting and calf-abutting regions. The top of the calf-abutting region is provided with a closure mechanism that enables the device to be secured to the calf of a patient, and the sole abutting region acts in conjunction with the shoe to support the foot of the patient.

Another previously proposed device is known colloquially as an "over foot" orthosis, meaning that the orthosis fits over the front (dorsal) aspect of the foot, rather than under it as in the abovementioned previously proposed devices, This "over foot" orthosis comprises a rigid plastics shell which is worn up against the skin, and which is secured around the calf by means of an appropriate securing band. The orthosis includes a stirrup which fits over the foot in the region of the instep to provide the patient with a resistance to plantarflexion.

All of the aforementioned orthoses provide the patient with a device which is capable of resisting plantarflexion. However, it is also the case that each of them has a number of attendant disadvantages.

To alleviate these problems, we have previously provided (see granted UK Patent No. 2330309) a sock-like structure which is formed of a resiliently flexible material - such as silicone for example. The sock-like structure, by virtue of the inherent resilience of the material from which it is made, provides a resistance to plantarflexion and also stores energy which can subsequently be released to assist dorsiflexion. The orthosis can be coloured to mimic the colour of the patient's' skin (and as such is cosmetically pleasing), can comfortably be worn in a normal off-the-shelf shoe, and need not be worn with a shoe in order to provide a beneficial effect.

The part of the sock-like structure which envelops the patient's ankle and lower leg in use includes an opening (to permit the user to put on the device), and in the preferred arrangement the opening is closed (to secure the orthosis in place on the foot of a patient) by means of respective parts of a mechanical hook and loop closure (such as velcro^{™}) which are embedded in the sock-like structure. Typically, one part of the closure is provided on the outside of the orthosis adjacent one side of the opening and the other part is provided on the inside surface of a tab extending from the other side of the opening, the closure on the tab being attachable to the closure adjacent the one side of the opening to close the opening, and secure the orthosis in place.

Our previous orthosis represented a quantum leap in the field and alleviated most (if not all) of the disadvantages mentioned above (the bulk of which had long been associated with previously proposed devices). Whilst this orthosis has proved to be extremely commercially successful, it remains the case that the process by which such devices are manufactured and fitted is relatively time consuming, and as a result these devices are more expensive to purchase than we would otherwise like.

To address this problem we have previously proposed, in United Kingdom Patent Application No. 2420716 for example, to provide a different arrangement for closing the opening in the part of the sock-like structure that envelops the patient's ankle and lower leg in use. Specifically, in the preferred embodiment described in this application, closure of the opening is achieved by configuring the part of the orthosis that envelops the lower leg and ankle of the patient so that it includes a second part that is arranged to overlap a first part when the orthosis is in a closed position fitted about the lower leg of the patient (the second part being moveable in a direction away from the first part to open the opening). One or more discrete closures are then fixedly attached (for example by a so-called speed rivet) to the second part and these closures can be wound round the lower leg of the patient and then secured to themselves to keep the opening closed in use.

One advantage of this arrangement is that by providing one or more separate closures, the embedding of velcro^{™} in a silicone body can be avoided, and hence the ankle-foot orthosis can more quickly and inexpensively be manufactured than the orthosis described in our previous UK patent GB2330309.

Whilst the orthosis of GB2420716 does greatly improve upon the orthosis described in GB2330309 (at least from the point of view of the time required to manufacture and fit the orthosis, and hence the cost of purchasing the orthosis), the process of making and fitting such orthoses is still relatively lengthy, and as a consequence devices of the type described in this application are still relatively expensive - particularly for those patients who do not have medical insurance or who are not eligible for state support.

On investigation we have identified that a principal reason for this is that as patients tend to suffer to differing degrees from foot-drop, the orthoses described in both GB2420716 and GB2330309 are necessarily bespoke products that are manufactured to address the particular requirements of each of the patients presenting and as such the manufacturing and fitting process for each orthosis is generally wholly patient specific.

Clearly, the ideal solution would be to propose a universal orthosis that is suitable for all patients irrespective of the size of the patient's foot and lower leg and the seriousness of the particular condition that they are suffering from. However, this is not a practical aim as patients inevitably have differently sized feet and lower legs, and tend to suffer to differing degrees from plantarflexion.

In light of the foregoing, an aim of the present invention is to address the problem of how to reduce the time taken to manufacture and fit an orthosis given the natural constraints of differing patient sizes and requirements.

EP0619102 discloses an ankle brace having a rigidifying and unitizing external shell comprising a unitary member made of a rigid material and including a leg encircling portion at least substantially encircling the lower leg of the wearer and ankle stays extending downwardly from the leg encircling portion over the ankle and down to the heel on both the medial and lateral sides of the leg.

US20040828895 discloses a foot orthosis includes a generally "L"-shaped splint having a generally upright leg-engaging section and a forwardly-extending foot support section with at least a portion of the splint being substantially transparent.

US5217431 discloses an orthopedic ankle brace is provided having a pliant boot that surrounds the ankle joint, as well as the foot and lower leg in the region thereof. The boot may incorporate means of applying compression to the ankle joint. Attached to the boot are a pair of adjustable tension straps vertically disposed about the ankle joint for restricting the mobility thereof.

US7101346 discloses an orthotic device that controls the (un)desired movements of the patient's defective lower limb. The device incorporates a footplate made of a rubber like elastomeric material, a proximal segment that encompasses the foot at the metatarsal phalengeal joints and the dorsal aspect of the foot, and a second proximal segment that encompasses the shin portion of the leg.

WO87/02885 discloses an ankle support with an underliner having multidirectional stretch which fits over the wearer's foot in the manner of a sock and extends to a point above the ankle. A non-stretch lateral strap or is secured to the underliner at a point below the ankle joint and extends upwardly to the top of the underliner, where it is inelastically secured, with provision being made for adjustment of its tension.

US2003233062 discloses an ankle brace for comfortably supporting an ankle of a wearer by inhibiting motion between a foot and lower leg of the wearer. The ankle brace employs a laminated, moisture-wicking material for leg and foot portions that improves wearability during athletic activities.

WO99/34757 discloses an ankle support for placement over a shoe that comprises a first sheet for forming a first side disposed over a shoe and a second sheet for forming a second side disposed over a shoe. The rear edge of the first sheet is attached to the rear edge of the second sheet. A first fastener part is disposed along the front edge of the first sheet and a second fastener part is disposed along the front edge of the second sheet for engaging the first fastener part to securely attach the front edge of the first sheet to the front edge of the second sheet

### Statement of Invention

In pursuit of this aim, a presently preferred embodiment of the invention provides a resiliently flexible structure that is formable into an ankle-foot orthosis that is configured to resist plantarflexion of a patient's foot, the resiliently flexible structure being as claimed in Claim 1.

Another related embodiment of the present invention relates to a method of fitting a resiliently flexible structure to the foot and lower leg of a patient to provide an ankle-foot orthosis for resisting plantarflexion of the patient's foot, the method being as claimed in Claim 13.

The principal advantage of these embodiments over the arrangements previously proposed is that the orthosis can more quickly be manufactured and fitted to a patient (as compared for example with the orthosis described in GB2330309). Specifically, by providing an arrangement where the first portion of the resiliently flexible structure can be fitted round the foot of a patient and then secured in place, it may no longer be necessary to cast each patient's foot before fitting. Instead one can simply select a suitable resiliently flexible structure from a previously manufactured stock of different resiliently flexible structures (each of which is suitable for patients whose feet fall within a relatively small range of foot sizes, for example up to three shoe sizes, and for whom the resistance to plantarflexion provided by the resiliently flexible structure is suitable for correcting the particular degree of plantarflexion that they experience) and then adjust that selected structure to provide an orthosis that fits the patient. It is also the case that manufacture of such a structure is generally easier to accomplish than manufacture of a bespoke orthosis (where greater care must be taken to ensure that all measurements taken for the patient's foot are faithfully reproduced), and hence the process by which such a structure is manufactured is generally faster than that for a bespoke orthosis.

As will be appreciated, by reducing the time required for the overall manufacturing and fitting process it is possible to reduce the sale price of orthoses according to the present invention as compared, for example, with orthoses of the type described in GB2330309 and GB2420716. In tests we have found that the time required to complete the overall manufacturing and fitting process described herein can be reduced by approximately forty percent as compared to the overall process time for an orthosis as described in GB2330309, and by approximately twenty-five percent as compared to the overall process time for an orthosis as described in GB2420716. We anticipate that by virtue of this reduction we will be able to sell orthoses of the type described herein for about forty percent of the cost of orthoses of the type described in GB2330309 and about seventy-five percent of the cost of orthoses of the type described in GB2420716.

The teachings of the present invention are well suited for use with orthoses as described in GB2330309 or GB2420716, but it will be appreciated that in the latter case the time savings resulting from the teachings of this invention combine with those provided by the teachings of GB2420716 to provide a particularly advantageous arrangement.

As described above, a principal advantage of this aspect of the invention is that fitting of the resiliently flexible structure to the patient can simply and quickly be achieved. Another advantage is that as the resiliently flexible structure is fitted to the patient (instead of being fitted to a cast of the patient's foot and lower leg) it is usually possible to correctly fit the structure at the first attempt. This contrasts with traditional methods where it is usual for the orthosis to be constructed on a cast which should be (but may not actually be) an accurate representation of the patient's foot and lower leg, and for the shape of the orthosis to then be adjusted when it is fitted to the patient.

Yet another presently preferred embodiment of the present invention relates to a method of manufacturing a resiliently flexible structure for fitting, in accordance with the method aforementioned, to the foot and lower leg of a patient to provide an ankle foot orthosis for resisting plantarflexion of the patient's foot, the method being as claimed in Claim 14.

Yet another embodiment of the invention relates to an ankle-foot orthosis formed from the resiliently flexible structure described herein, the ankle-foot orthosis being as claimed in Claim 15.

Preferred features of each of these embodiments are set out in the accompanying claims and elsewhere in the following description. Further objects, features and advantages of embodiments of the present invention will also be apparent from the detailed description that follows.

### Brief Description of the Drawings

Various preferred embodiments of the invention will now be described, by way of illustrative example only, with reference to the accompanying drawings, in which:
Fig. 1 is a schematic rear perspective view of an orthosis in accordance with a preferred embodiment of the present invention in a closed configuration, viewed from the instep side of a patient's right foot;
Fig. 2 is a rear perspective view of the orthosis shown in Fig. 1 in a partly open configuration;
Fig. 3 is a rear perspective view of the orthosis depicted in Figs. 1 and 2 in a fully opened configuration;
Fig. 4 is a rear perspective view of a resiliently flexible structure prior to having been fitted to a patient's foot;
Figs. 5 to 7 illustrate a method of fitting a resiliently flexible structure of the type depicted in Fig. 4 to a patient, in this instance to a patient's left foot;
Fig 8 is a rear perspective view of an orthosis according to a second embodiment of the invention in a closed configuration;
Fig. 9 is a rear perspective view of the orthosis depicted in Fig. 7 in an open configuration; and
Figs. 10 to 12 are schematic representations of alternative closure mechanisms.

### Detailed Description of Preferred Embodiments

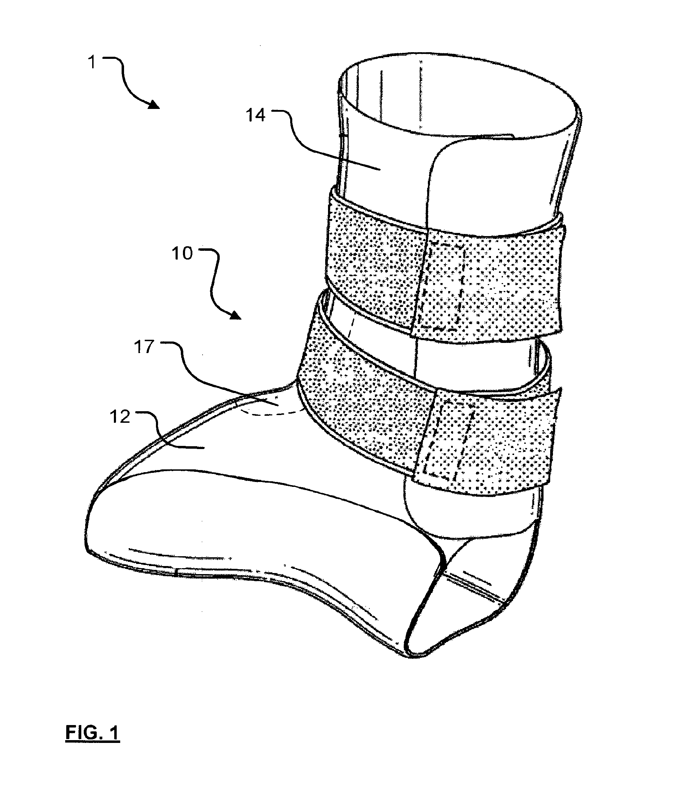

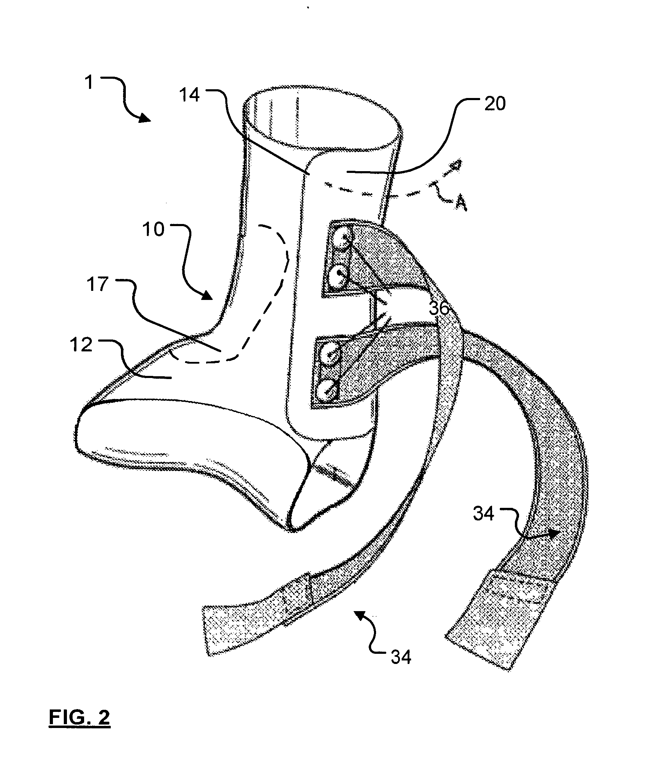

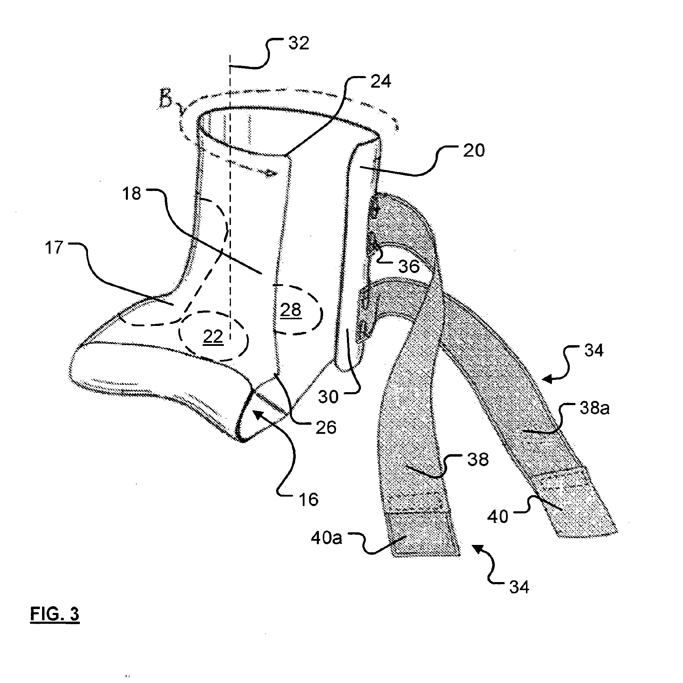

Fig. 1 is a perspective view of an embodiment of the invention in a closed configuration. The orthosis 1 depicted in Fig. 1 is shown as it would appear after it has been fitted to a patient and whilst it is being worn by a patient (the patient's foot, ankle and lower leg having been omitted for clarity). The view provided in Fig. 1 is of the instep side of the patient's right foot. It worth noting that the orthosis is sufficiently resilient to keep its three dimensional shape (for example as shown in Figs. 2 and 3) when not being worn by the patient.

The orthosis comprises an orthotic structure 10 which is comprised of a first tubular portion 12 and a second tubular portion 14. The first and second portions are contiguous, and in most instances the second tubular portion 14 will have been integrally formed with the first portion 12. The first and second portions are set at an angle to one another to provide, at least in use, a generally L- shaped internal cavity 16 (Fig. 3) which is sized such that the orthosis accepts and fits closely about the foot, ankle and at least a portion of the lower leg of a patient (not shown) in use.

In a highly preferred arrangement the sock-like structure is formed not so that the first portion is set perpendicular to the second, but so that the structure exhibits approximately 3 to 10, preferably 5, degrees of dorsiflexion. In the preferred arrangement this dorsiflexion drops to zero when the orthosis is being worn by a patient and the weight of their foot is applied to the orthosis during the swing-through phase of their gait.

The orthotic structure 10 may be described as being generally "sock-like", or in other words akin to a sock, in that when worn by a patient the second portion 14 envelops a portion of the patient's lower leg including the medial malleolus (the inside of the ankle) and the lateral malleolus (the outside of the ankle), and the first portion 12 envelops at least a portion of the plantar aspect of the foot (the sole of the foot) and at least a portion of the dorsal aspect of the foot (the top/front of the foot).

In the preferred arrangement (as depicted) the orthotic structure is configured so that the patient's toes and calcaneum (heel) are exposed. This is because we have found that an orthosis which exposes the heel and toes is significantly more comfortable for the patient to wear, whilst also giving the patient a greater sense of confidence when walking (particularly when barefoot) on a given surface due to the fact that their toes and heel can grip that surface. It will be appreciated, however, that the orthosis may instead be configured to envelop the toes and/or heel if desired.

As a further alternative for those patients with particularly sensitive ankles, the first and second portions may be configured so that the walls of the first and second portions are thinned or include apertures in those regions of the structure which would normally overlie the medial and lateral malleolae.

The resilience of the orthotic structure 10 is chosen, and may be varied, in dependence upon the degree to which the patient suffers from plantarflexion. In particular, the resilience of the orthotic structure 10 is chosen to provide a resistance to plantarflexion that is sufficient for correcting the particular degree of excessive plantarflexion experienced by the patient who is going to be using the orthosis.

Variations in the resilience of a given orthotic structure 10 (as may be required for patients experiencing a lesser or greater degree of excessive plantarflexion) may be effected by changing the material from which the orthotic structure is formed, by changing the thickness of the orthotic structure, or by incorporating reinforcing means - such as a resilient rib - into the orthosis (or indeed by means of a combination of these).

As an illustrative example, the orthotic structure could include reinforcing means in the form of a resilient rib 17 (shown in ghost in Fig. 1) that extends partway along the dorsal aspect of the foot. The rib 17 could be formed integrally with the orthosis, or could be removably insertable into a pocket provided on the dorsal aspect of the orthotic structure. The latter arrangement would be particularly useful in that it would allow for the stiffness of the rib and/or the angle of support to be changed if desired (by inserting differently shaped ribs). The rib may be of the same material as the remainder of the orthotic structure 10, or may be of a different - preferably more resilient - material such as plastics, metal or carbon fibre.

The first 12 and second 14 portions are formed of a resiliently flexible material, and in the preferred embodiment this material is a silicone elastomer. Preferably the silicone elastomer has a Shore A durometer of 30 to 80, preferably of 30 to 70, and most preferably of 50 or 70.

The use of a resilient material for the orthotic structure 10 (and optionally for the rib, if provided) is a fundamental departure from "under foot" or "over foot" devices of the type described above. These previously proposed devices recommended the use of a non-resilient, i.e. rigid, material. The primary advantage of using a resilient material as opposed to a rigid material is that the material can flex to store energy during certain phases of the patient's gait, and then release that energy during other phases of the patient's gait (in particular the swing-through phase) to thereby actively assist the walking process as a whole, and dorsiflection in particular.

Dorsiflexion and plantarflexion of a foot is predominantly controlled by the tibialis anterior muscle and tendon, and the structure of the orthosis functions to assist the operation of this muscle - in particular for those patients who experience persisting foot drop resulting from a neurological impairment caused, for example, by trauma, disease or genetics. This bio-mechanical function of the device of the invention is fundamentally different to so-called athletic support stockings, for example those of the tubigrip^{™} type, which provide no means for assisting the operation of the tibialis anterior muscle and tendon (to resist plantarflexion and assist dorsiflexion of a patient's foot), and are instead wholly concerned with resisting abnormal lateral movement of the foot.

As aforementioned, the orthotic structure 10 is closely fitted to the foot and lower leg of the patient (in a manner that is later described) and as such may be configured to provide a compressive force to the foot and lower leg of the patient. In a preferred embodiment, the compressive force may be tailored to help treat other conditions such as the effects and symptoms of venous insufficiency, for example varicose veins. It is also conceivable for different regions of the orthotic structure to provide different compressive forces. For example, the orthotic structure may exert a greater compressive force on the foot of the patient than on the ankle in order to provide a pumping effect to assist blood flow to and from the foot.

Referring now to Figs. 2 & 3, the second tubular portion 14 of the orthotic structure is configured so that it can be opened to permit the orthosis to be put on by a patient. In this embodiment opening of the second tubular portion 14 is achieved by configuring the second tubular portion so that it comprises a second part 20 that is arranged to overlap a first part 18 when the orthosis is in a closed position fitted about the lower leg of the patient - the second part 20 being moveable (in a direction A indicated generally in Fig. 2) away from the first part 18 to open the second tubular section 14.

In the preferred arrangement, as depicted in Fig. 3, the first part 18 comprises a part of the second tubular section 14 which extends generally from a part 22 of the orthotic structure 10 which overlies the medial malleolus when the orthosis is worn by a patient, and terminates at a line running longitudinally from an uppermost edge 24 of the second tubular section 14 (i.e. that edge which is in the vicinity of the patient's lower leg in use) to a lowermost edge 26 of the second tubular section 14 (i.e. that edge which is in the vicinity of the patient's calcaneum in use) generally midway between the part 22 of the second tubular section that is arranged to overlie the patient's medial malleolus in use and a part 28 of the second tubular section that is arranged to overlie the patient's lateral malleolus in use. The second part 20 comprises a part of the second tubular section 14 which extends generally from the part 28 of the orthotic structure 10 which overlies the patient's lateral malleolus to form a tab portion 30 which overlaps the first part 18 when the second tubular section 14 is closed. In the preferred arrangement the tab portion 30 overlaps the first part 18 (when the second tubular section 14 is closed) to an extent whereby the tab portion 30 terminates at an axial line 32 running longitudinally down the second tubular section and terminating at or about that portion 22 of the orthosis which overlies the patient's medial malleolus in use.

As depicted, the orthosis further comprises two closures 34 which, in this embodiment, are in the form of two closure strips that are each fixedly attached to the aforementioned tab portion 30 and which can be used - in a manner described in detail below - to secure the second tubular portion 14 in the aforementioned closed position about the lower leg of the patient. In the preferred arrangement two closure members are provided, but it will be appreciated that a single closure member or more than two closure members may be provided if desired.

In the preferred embodiment, the closure strips are each fixedly attached to the tab portion by means of tubular double-headed rivets 36, also known as "speed rivets", (or equivalent fixings) the like of which are available from Evans and Evans (Unit 24, Red Lion Business Park, Red Lion Road, Tolworth, Surbiton, Surrey, UK), or Algeos (Sheridan House, Bridge Industrial Estate, Speke Hall Road, Liverpool, L249HB United Kingdom). Whilst it is undoubtedly the case that a variety of different fixings will be immediately evident to those persons skilled in the art, speed rivets are nevertheless preferred as they permit the attachment of the closure to the orthosis to be accomplished quickly.

In the preferred embodiment where the tab portion 30 of the second tubular section 14 overlaps the first part 18, it is preferred that the closures 34 are attached to the tab portion 30 in such a manner that the fixings 36 also overlap the aforementioned first part 18 of the second tubular section 14. This is highly advantageous as it means that the fixings then do not bear on the patient's lower leg (where they could cause discomfort) but instead bear upon the first part of the second tubular section.

In the preferred arrangement, the closures each comprise a first strip of material 38 carrying on the outside 38a thereof (i.e. the side facing away from the second tubular section) one part of a mechanical hook and loop securing system (such as velcro). A second strip of material 40 carrying on the inside 40a thereof (i.e. the side facing towards the second tubular section) the other part of the mechanical hook and loop securing system is attached (for example sown onto) the free end of the first strip of material 38. In a highly preferred arrangement the first strip of material 38 carries a set of hoops (and the second strip 40 carries the hooks) so that the closures do not interfere with any clothes that the patient might be wearing.

As mentioned above, the closures are provided to enable the second tubular section to be secured in the aforementioned closed position depicted in Fig. 1. To this end, the closures can be wound (in the direction B indicated in Fig. 3 - i.e. in a continuation of the direction A (Fig. 2) in which the tab portion 30 may be moved to open the second tubular section 14) round the periphery of the second tubular section 14 until the hooks or loops on the second strip of material 40 overlie the loops or hooks on the first strip of material 38, whereupon the second strip of material 40 can be secured to the first 38 to secure the second tubular section 14 in the closed position.

As an alternative, the closures 34 could be affixed to the tab section 30 in such a way that they are wound in the opposite direction (i.e. in a direction which would move the tab portion to close the second tubular portion) and then secured as aforementioned. However, such an arrangement is less preferred as pulling the closures in a direction which acts to close the tubular section 14 may encourage the patient to overtighten the second tubular section about their lower leg, perhaps to a point where the second tubular section is secured so tightly that it impacts adversely on the circulation in the patients lower leg and foot. This is particularly the case for those patients who have suffered nerve damage and might not immediately be aware that the orthosis has been overtightened.

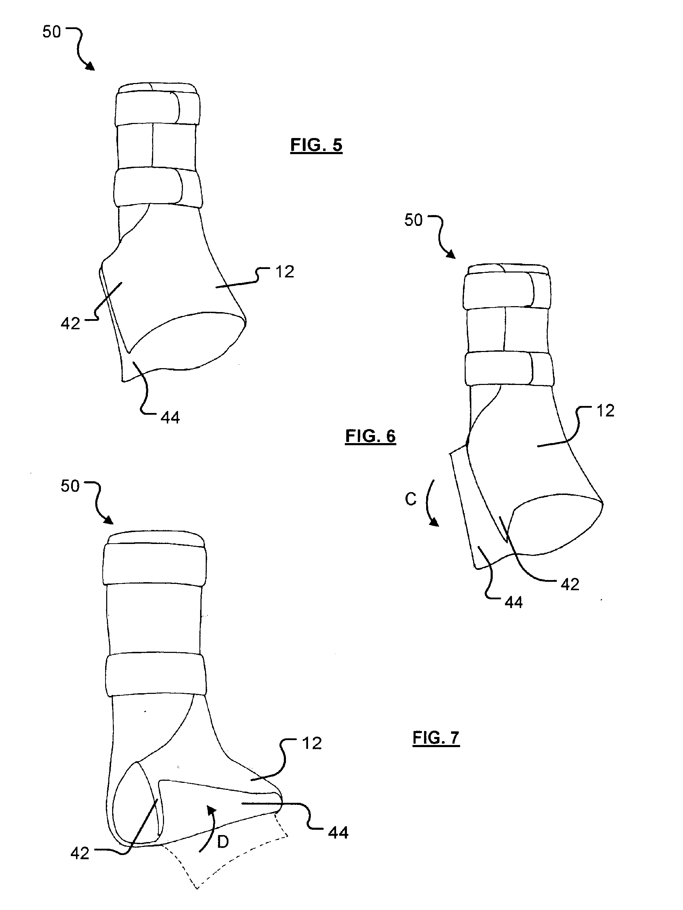

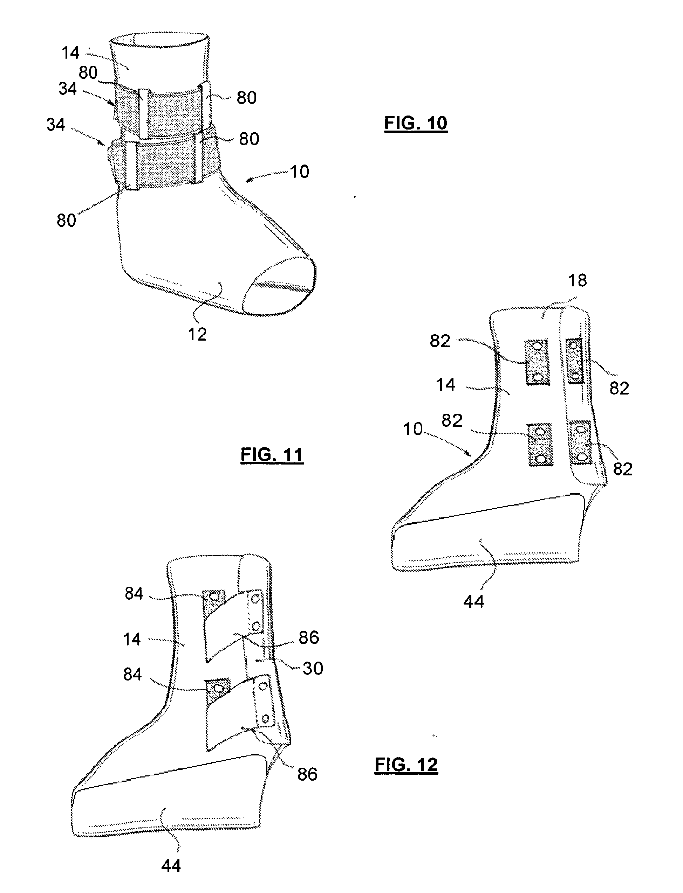

Whilst the above arrangement is preferred, it will be apparent that a number of other alternatives are possible. For example, it is not necessary for the closures to be attached to the second tubular section. The closures could be detachable from the second tubular member, and simply secured around it. The second tubular section 14, for example as is depicted schematically in Fig. 6 of GB2420716 and Fig. 10 of the present application, could also be provided with a number of hoops 80 through which fully detachable closures 34 could be threaded and secured (in a manner akin to threading a belt through belt hoops on a pair of trousers).

As a further alternative, depicted schematically in Fig. 7 of GB2420716 and Fig. 11 of the present application, the closures could comprise a piece of material (not shown) that is fully detachable from the orthosis and which carries hooks or loops of a mechanical hook and loop engagement mechanism. In this arrangement, again as is described in detail in GB2420716, the orthosis has two strips of material 82 per closure that are fixedly attached (for example by means of speed rivets or equivalent fixings) to the second tubular section - the first being attached to the outside of the aforementioned tab portion and the second being attached to the outside of a region of the aforementioned first part of the second tubular section which is not overlapped by the tab portion when the second tubular section is closed. Each of the strips of material carry loops or hooks of the mechanical hook and loop engagement mechanism, so that the detachable piece of material can be secured to each of the two attached strips of material to hold the second tubular section in the closed position.

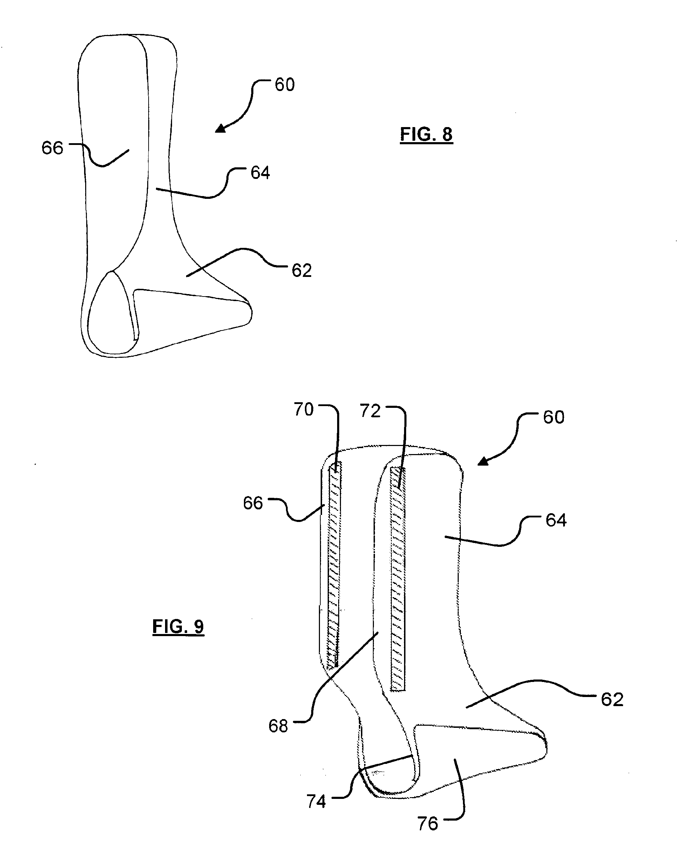

As a yet further alternative (depicted schematically in Fig. 8 of GB2420716 and Fig. 12 of the present application), a strip of material 84 (carrying hooks or loops of a mechanical hook and loop engagement mechanism on its outward face) may be attached to the outside of a region of the second tubular section 14 which is not overlapped by the tab portion 30 when the second tubular section 14 is closed. A second strip of material 86, fixedly attached at one end to the tab portion 30 and carrying on its underside (i.e. the side facing the second tubular section) loops or hooks of the mechanical engagement mechanism may then be secured to the strip of material fixedly attached to the second tubular section to secure the second tubular section in the aforementioned closed position.

In another modification of the preferred embodiment, the closure 34 does not necessarily need to comprise two strips of material (38, 40) attached to one another. Rather, the closure could instead comprise a single strip of material provided with hooks or loops of the mechanical engagement mechanism on one side, and loops or hooks of the mechanism provided on the other.

As a further modification, the mechanical hook and loop closure mechanism could be replaced by another type of fixing - such as press studs for example.

In a further alternative arrangement not depicted in the drawings, the second tubular portion could be longitudinally split, with respective edges of the split being joined by a section of elastic material - the elastic material being sown or otherwise affixed to the resilient material of the second tubular portion, and permitting the second tubular section 14 to be opened for the insertion of a patient's foot and lower leg. Any of the abovementioned closure members could then be provided to secure the second tubular section around the patient's lower leg (suitable modifications being made to account for the absence of a tab portion).

It will also be apparent to those persons skilled in the art that the tab portion 30 of the second tubular portion 14 need not overlap the first part 18. The second tubular portion 14 could simply comprise a longitudinal slit, one or more closure members being provided to bring opposed edges of the slit towards one another to close the slit and secure the second tubular section about the lower leg of the patient. Such an arrangement, whilst eminently possible, is less preferred as the fixings used to attach the closure member(s) to the orthosis would then be in contact with the patient's lower leg.

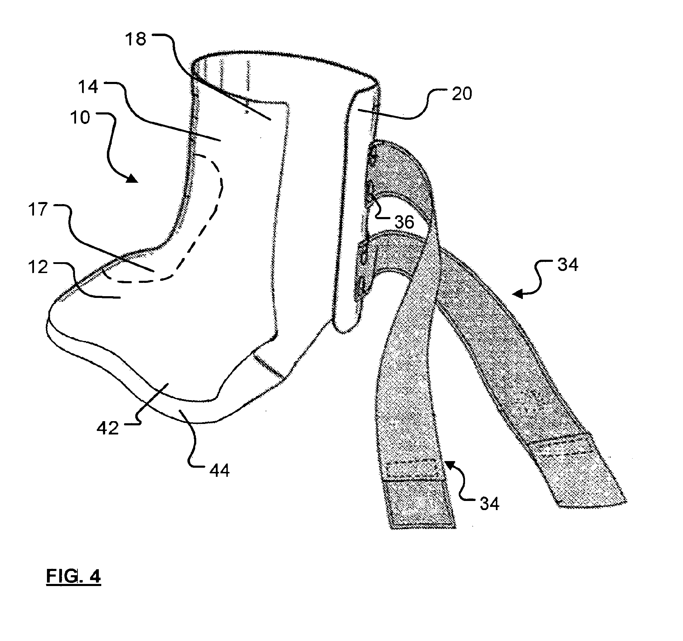

Referring again to Fig. 1, the first portion 12 of the fitted finished orthosis comprises a tube formed by overlapping a first region 42 and a second region 44 (best viewed in Fig. 4) of the first portion 12 of the orthotic structure 10, and then permanently fixing the overlapped regions together. In the preferred embodiment the first and second regions 42, 44 are adhered to one another, and in the context of an orthotic structure manufactured of silicone, the adhesive selected to adhere these portions is preferably a silicone adhesive such as a so-called room temperature vulcanising adhesive, of which silicone adhesive I-400310 provided by Össur, Grjothals 5, 110 Reykjavik, Iceland is one particularly suitable example.

Whilst it is preferred, for simplicity, for the first and second regions to be permanently adhered one to the other, it will be apparent to persons skilled in the art that the two regions may otherwise be permanently fixed together. For example, depending on the material chosen for the orthotic structure, the first and second regions may be affixed by means of a heat treating or welding process (for example by ultrasonic welding), by means of a different type of adhesive, or by any other means known to persons skilled in the art.

As will immediately be appreciated by persons skilled in the art, it is of no consequence whether the first region overlies the second, or the second overlies the first. It is also of no consequence whether the overlap is provided on the instep side of the orthosis (i.e. that side of the orthosis which covers the instep of the patient's foot in use) or the outside of the orthosis (i.e. that side of the orthosis which covers the outside (i.e. the side proximate the patient's little toe) of the patient's foot in use. It is even conceivable for the overlap to occur on the dorsal or plantar aspect of the orthosis, but as will be appreciated the orthosis can more simply be constructed if the overlap is provided on the instep side or outside of the first portion 12.

As is later described, the degree to which the first and second portions overlap is adjusted during fitting of the orthosis to the patient so that the first tubular portion 12 forms a close fit to the foot of the patient. The "adjustable" nature of the first tubular portion 12 is advantageous as it means that the orthotic structure need no longer be manufactured with a bespoke first portion, and hence that it may no longer be necessary to cast or accurately measure a patient's foot (which processes takes a considerable amount of time). It is also the case that as the shape of the first portion need not be formed as accurately as, for example, the first portion of the orthoses described in GB2330309 and GB2420716, the orthosis can more quickly be manufactured than previously proposed orthoses, and furthermore that adjustments to the shape of the first portion can quickly be made in most instances without having to remove material from or add material to a bespoke first portion (as previously occurred with the orthoses described in the abovementioned GB patent applications).

Referring now to Fig. 4 of the accompanying drawings, there is depicted a resiliently flexible structure 50 that has not yet been fitted to a patient. It is envisaged that many such devices will be manufactured, with each device being suitable for fitting to patients with a range of foot sizes (for example a range of up to 3 to 4 shoe sizes). Each structure will also have a resilience that is appropriate for correction of a range of severities of foot-drop. For example, resiliently flexible structures may be manufactured to have resiliences suitable for correction of mild, moderate or severe foot drop, and structures of these resiliences may be manufactured to fit shoe sizes 3-6, 6-9 or 9-12. It is apparent, therefore, that in this envisaged implementation a collection of nine resiliently flexible structures would provide for the needs of any adult patient suffering from foot-drop. Similar arrangements can be made for child patients, and the ranges covered by any given resiliently flexible structure can be expanded (so that a smaller number of structures are required for full coverage of patients presenting) or reduced as desired.

The resiliently flexible structure is manufactured to include a closure 34 (in this instance two closure strips), and the first 42 and second 44 regions of the first portion co-operate to define an opening 46 that will later be closed when the resiliently flexible structure is fitted to a patient to form an orthosis.

This fitting process will now be described with reference to Figs. 5 to 7 of the drawings (in which a resiliently flexible structure suitable for fitting to a patient's left foot is shown - the patient's foot having been omitted for clarity). In a first step of the fitting process, a patient presenting with foot-drop is assessed to determine their shoe size and the extent to which they suffer from foot-drop. Once this information has been obtained, a resiliently flexible structure with a suitable resilience for correction of the severity of foot drop experienced by the patient, and of a suitable size for their shoe size, is selected. The selected resiliently flexible structure is then put on by the patient, as shown in Fig. 5, and the second tubular portion 14 is secured about the patient's lower leg by means of the closure 34.

In the next step of the process depicted in Fig. 6, the first region 42 of the first portion 12 is moved towards the patient's foot generally in the direction indicated by arrow C, following which the second region 44 of the first portion 12 is moved in a direction indicated by arrow D (as indicated in Fig. 7) to overlap the first region 42 until the first portion 12 is closely fitted to the patient's foot. The second region 44 is then fixed, for example adhered, to the first region to provide a first portion 12 that is in the form of a tube that is closely fitted to the patient's foot.

Fixing of the second region 44 to the first region 42 may be accomplished in situ, or in a particularly preferred arrangement the degree to which the second region overlaps the first region is marked on the resiliently flexible structure following which the patient takes off the resiliently flexible structure so that the second region can be fixed to the first without the patient being present. To accomplish this, once the resiliently flexible structure has been taken off, a suitable adhesive for example is applied to the first and/or second regions, following which the second region is moved to overlap the first region to the extent previously marked and held in place until the adhesive has cured.

As will be appreciated by persons skilled in the art, adjusting a resiliently flexible structure to fit in the manner aforementioned is significantly easier (and hence quicker) than manufacturing a bespoke orthosis for each presenting patient.

Whilst it is the case that the benefits provided by the present invention are particularly apparent when embodied in a device of the type shown in Figs. 1 to 7, the teachings of the present invention can also be incorporated into an orthosis of the type described and depicted in our previous UK patent, GB2330309.

Figs. 8 and 9 are rear perspective views, in closed and open configurations respectively, of an orthosis 60 of the type described in this UK patent. The orthosis comprises, as in the preferred embodiment of this invention, first 62 and second 64 contiguous tubular portions set at an angle to one another. Further details of this orthosis are provided in GB2330309.

As shown in Fig. 9, the second tubular portion 64 includes an insertion slit that is closed by overlapping first 66 and second 68 regions of the second tubular portion, and then securing those regions to one another by means, in the preferred embodiment, of a two-part mechanical fastener, for example velcro^{™} - respective parts 70, 72 of which are embedded in the silicone of the second tubular portion 64.

As shown in Figs. 8 and 9, in the orthosis 60 of this embodiment the first portion 62 of this orthosis 60 has been formed into a tube that closely fits the foot of the patient by overlapping first 74 and second 76 regions of the first portion and permanently securing those regions together in the manner aforementioned.

The devices described herein may be coloured so that they can be matched to the skin colour of the patient (as can the closure member(s)), and may be provided in a variety of different sizes and shapes. The resiliently flexible structure could be manufactured by injection moulding (or any other suitable process), but in the preferred embodiment it is manufactured by milling (as described below) and subsequently building up layers of silicone elastomer material upon a suitably sized generic three dimensional representation of a foot and lower leg.

The resiliently flexible structure is manufactured from a resiliently flexible silicone elastomer materials.

The performance of the devices described herein is expected to be at least on a par with that provided by our previously proposed device described in granted UK Patent No. 2330309. This device was found to provide a considerable improvement not only to the degree of plantarflexion, but also to the walking speed and the effort involved in walking (known as the Physiological Cost Index or PCI) of a group of patients. At an initial point in the study it was found that the orthosis provided an increase of roughly 10% in walking speed and a reduction of roughly 2% in the PCI.

At the end of the study, roughly six months later, it was found that that same group of patients experienced an increase in walking speed of roughly 20% and a reduction of roughly 32% in the PCI as compared to when they were initially without the orthosis.

It is anticipated that the orthosis of the present invention will provide similar, and hopefully, better results.

We have also found that the orthoses of the present invention improve the patient's proprioception. In particular, when not wearing an orthosis as herein described, we have noted that patients often tend to stumble when walking. However, when the orthosis is worn, the pressure exerted by the orthosis on the skin receptors sends a message to the brain that helps the patient determine where the foot is in space. This in turn helps the patients to walk faster and avoid stumbling.

It will be understood from the above that the orthoses herein described provide an effective means to tackle the problem of plantarflexion. Advantageously, and in addition to this function, the orthoses herein described can significantly augment dorsiflexion during the swing-through phase of a patient's gait cycle. The principal reason for this is believed to be that the orthotic structure (and resilient rib, if provided) store energy when compressed, and this energy is released during the swing through phase of the patient's gait cycle. It is anticipated, therefore, that patients will not only find that the orthoses of the embodiments tackle the problem of plantarflexion but also actively assist the walking process.

## Claims

1. A resiliently flexible structure (50) to be formed into an ankle-foot orthosis (10; 60) that is configured to resist plantarflexion of a patient's foot, the resiliently flexible structure (50) comprising a first portion (12; 62) and a second portion (14; 64) set at an angle to said first portion (12; 62), wherein:
said first portion (12; 62) comprises first and second regions (42, 44; 74, 76) that cooperate to define an opening and are configured and arranged so as to be overlappable and permanently securable to one another to form a tube that fits closely about the patient's foot;
said second portion (14; 64) is formed as a split tube that is adapted to be opened for insertion of the patient's foot into the tube formed by said first portion (12; 62), and for insertion of at least a portion of the patient's lower leg into said second portion (14; 64), and
said resiliently flexible structure (50) further comprises a closure (34; 70, 72; 82; 84, 86) for securing said second portion (14; 64) in a closed position in which the second portion (14; 64) closely fits about the lower leg of the patient in use; **characterised in that** the resiliently flexible structure (50) is of silicone elastomer and has a resilience that is chosen to be sufficient to provide a resistance to flexure that is appropriate for correction of the particular degree of plantarflexion experienced by the patient.

2. A structure according to Claim 1, wherein the second tubular portion (14, 64) is longitudinally split to define an insertion slit, first and second edge portions of the slit being configured to lie substantially adjacent one another when the second tubular portion (14; 64) is in the closed position.

3. A structure according to Claim 1, wherein the second tubular portion (14, 64) comprises first and second edge portions (18, 20, 30; 66, 68), said second edge portion (20, 30; 66) being separable from the first portion to permit the second tubular portion (14; 64) to be opened and being configured to overlie the first edge portion (18; 68) when the second tubular portion (14; 64) is in the closed position.

4. A structure according to Claim 2 or 3, wherein the closure (34; 70; 72; 82; 84, 86) is fixedly attached to the orthotic structure.

5. A structure according to Claim 4, wherein the closure comprises a first part (84) fixedly attached to said second tubular portion (14), and a second part (86) fixedly attached to the second edge portion (30), at least a free end of the second part (86) being securable to the first part (84) to secure the second tubular portion (14) in said closed position.

6. A structure according to Claim 5, wherein said first part and at least a portion of said second part include appropriate complementary parts of a mechanical hook and loop securing system to permit the second part to be secured to said first part.

7. A structure according to Claim 4 when dependent on Claim 3, wherein the closure comprises a strap (34) fixedly attached at one end to said second edge portion (30) by means of a fixing (36), said strap (34) being configured to be passed round the entire circumference of the second tubular portion (14) before being secured to itself to urge the second tubular portion (14) towards the closed position, the arrangement being such that the fixing (36) bears on said first edge portion (18) when the second tubular portion (14) is in the closed position and not on the patient's lower leg.

8. A structure according to Claim 2 or 3, wherein the closure is at least partly separable from the resiliently flexible structure.

9. A structure according to Claim 8 when dependent on Claim 2, wherein said closure comprises first and second closure parts (82) fixedly attached to respective ones of said first and second edge portions (18, 20), and a removable closure strip attachable to both of said first and second closure parts (82) to secure the second tubular section (14) in said closed position.

10. A structure according to Claim 8 when dependent on Claim 3, wherein said closure comprises a first closure part (82) fixedly attached to said second tubular section (14), a second closure part (82) fixedly attached to the second edge portion (20), and a removable closure strip attachable to both of said first and second closure parts (82) to secure the second tubular section (14) in said closed position.

11. A structure according to Claim 3, wherein said closure comprises a first closure part (72) secured to an external surface of said first edge portion (68) and a second closure part (70) secured to an internal surface of said second edge portion (66), said first and second closure parts (70, 72) being securable together when said second edge portion (66) overlies the first edge portion (68) to retain said second tubular portion (64) in said closed position.

12. A structure according to any preceding claim, wherein the resiliently flexible structure (50) is of a silicone elastomer having a Shore A durometer of 30 to 80.

13. A method of fitting a resiliently flexible structure (50) to the foot and lower leg of a patient to provide an ankle-foot orthosis (10; 60) for resisting plantarflexion of the patient's foot, the method comprising:
determining the size of the patient's foot, and a degree to which the patient suffers from foot plantarflexion;
selecting a resiliently flexible structure (50) of silicone elastomer whose size is suitable for fitting to a patient having a foot of the determined size and whose resilience to plantarflexion is appropriate for correction of the determined degree to which the patient suffers from foot plantarflexion;
inserting the patient's foot and at least a portion of the patient's lower leg into the resiliently flexible structure (50);
overlapping first and second regions (42, 44; 74, 76) of a first portion (12; 62) of the resiliently flexible structure (50) into which said patient's foot has been inserted until said first portion (12; 62) closely fits about the patient's foot; and
permanently securing said first and second regions (42; 44; 74; 76) together.

14. A method of manufacturing a resiliently flexible structure (50) for fitting, in accordance with the method claimed in Claim 13, to the foot and lower leg of a patient to provide an ankle foot orthosis (10; 60) for resisting plantarflexion of the patient's foot, the method comprising:
preparing a stock of resiliently flexible silicone elastomer that has a resilience, at least when cured, which is appropriate for resisting the degree of plantarflexion experienced by a particular patient type;
applying the resiliently flexible silicone elastomer to a three-dimensional representation of a foot and lower leg for a particular patient type to form a resiliently flexible silicone elastomer structure (50) that comprises a first portion (12; 62) and a second tubular portion (14; 64) set at an angle to said first portion (12; 62), wherein
said first portion (12; 62) is configured to include first and second regions (42; 44; 74; 76) that cooperate to define an opening and are capable of being overlapped and being permanently secured to one another to form a tube, and
said second tubular portion (14; 64) is capable of being opened for insertion of a patient's foot into the tube formed by said first portion (12; 62), and for insertion of at least a portion of the patient's lower leg into said second portion (14; 64);
attaching a closure (34; 66, 68; 82; 84, 86) to said second portion (14; 64), said closure (34; 66, 68; 82; 84, 86) being operable to secure said second portion (14; 64) in a closed position in which the second tubular portion (14; 64) closely fits about the lower leg of a patient; and
allowing the resiliently flexible silicone elastomer to cure.

15. An ankle-foot orthosis (10; 60) formed from the resiliently flexible structure (50) of any of Claims 1 to 12, wherein: said first and second regions (42, 44; 74, 76) of said first portion (12; 62) overlap and are permanently secured to one another to form a tube that fits closely about the patient's foot.

## Patentansprüche

1. Belastbar flexible Struktur (50), die in eine Fußgelenk-Fuß-Orthese (10; 60) geformt werden soll, die derart konfiguriert ist, um einer Plantarflexion des Fußes eines Patienten zu widerstehen, wobei die belastbar flexible Struktur (50) einen ersten Abschnitt (12; 62) und einen zweiten Abschnitt (14; 64) umfasst, der in einem Winkel zum ersten Abschnitt (12; 62) steht, wobei:
der genannte erste Abschnitt (12; 62) erste und zweite Regionen (42, 44; 74, 76) umfasst, die derart miteinander kooperieren, um eine Öffnung zu definieren und die derart konfiguriert und angeordnet sind, um zu überlappen und permanent aneinander gesichert werden, um einen Schlauch zu bilden, der dicht um den Fuß des Patienten passt;
der zweite Abschnitt (14; 64) als ein gespaltener Schlauch geformt ist, der derart adaptiert ist, um für ein Einsetzen des Fußes des Patienten in den durch den ersten Abschnitt (12; 62) gebildeten Schlauch und für ein Einsetzen wenigstens eines Teils des unteren Beins des Patienten in den zweiten Abschnitt (14; 64) geöffnet werden zu können, und
die belastbar flexible Struktur (50) ferner einen Verschluss (34; 70, 72; 82; 84, 86) zum Sichern des zweiten Abschnitts (14; 64) in einer geschlossenen Position umfasst, in welcher der zweite Abschnitt (14; 64) dicht um das untere Bein des Patienten während dem Gebrauch passt; dadurch charakterisiert, dass die belastbar flexible Struktur (50) aus Silikonelastomer besteht und eine Belastbarkeit aufweist, die derart ausgewählt ist, um ausreichend zur Bereitstellung eines Widerstands gegen die Flexur ist, die für die Korrektur des bestimmten Ausmaßes der vom Patienten erfahrenen Plantarflexion geeignet ist.

2. Struktur nach Anspruch 1, wobei der zweite schlauchförmige Abschnitt (14, 64) längs aufgespalten ist, um einen Einsatzschlitz zu definieren, wobei die ersten und zweiten Kantenabschnitte des Schlitzes derart konfiguriert sind, um im Wesentlichen nebeneinander zu liegen, wenn sich der zweite schlauchförmige Abschnitt (14; 64) in der geschlossenen Position befindet.

3. Struktur nach Anspruch 1, wobei der zweite schlauchförmige Abschnitt (14, 64) erste und zweite Kantenabschnitte (18, 20, 30; 66, 68) umfasst, wobei der zweite Kantenabschnitt (20, 30; 66) vom ersten Abschnitt getrennt werden kann, um es dem zweiten schlauchförmigen Abschnitt (14; 64) zu ermöglichen, geöffnet zu werden und derart konfiguriert ist, um auf dem ersten Kantenabschnitt (18; 68) aufzuliegen, wenn sich der zweite schlauchförmige Abschnitt (14; 64) in der geschlossenen Position befindet.

4. Struktur nach Anspruch 2 oder 3, wobei der Verschluss (34; 70; 72; 82; 84, 86) starr an der Orthesenstruktur befestigt ist.

5. Struktur nach Anspruch 4, wobei der Verschluss einen ersten Teil (84) umfasst, der starr am zweiten schlauchförmigen Abschnitt (14) befestigt ist und einen zweiten Teil (86), der starr am zweiten Kantenabschnitt (30) befestigt ist, wobei wenigstens ein freies Ende des zweiten Teils (86) am ersten Teil (84) gesichert werden kann, um den zweiten schlauchförmigen Abschnitt (14) in der genannten geschlossenen Position zu sichern.

6. Struktur nach Anspruch 5, wobei der genannte erste Teil und wenigstens ein Abschnitt des genannten zweiten Teils entsprechende komplementäre Teile eines mechanischen Hakens und ein Schlaufensicherungssystem enthalten, um es dem zweiten Teil zu ermöglichen, am genannten ersten Teil gesichert zu werden.

7. Struktur nach Anspruch 4, wenn abhängig von Anspruch 3, wobei der Verschluss einen Gurt (34) umfasst, der starr an einem Ende des genannten zweiten Kantenabschnitts (30) mittels eines Fixiermittels (36) befestigt ist, wobei der Gurt (34) derart konfiguriert ist, dass er um den gesamten Umfang des zweiten schlauchförmigen Abschnitts (14) herum geführt wird, bevor dieser an sich selbst gesichert wird, um den zweiten schlauchförmigen Abschnitt (14) zur geschlossenen Position hin zu drängen, wobei die Anordnung derart gestaltet ist, dass das Fixiermittel (36) auf dem genannten ersten Kantenabschnitt (18) aufliegt, wenn sich der zweite schlauchförmige Abschnitt (14) in der geschlossenen Position befindet, und nicht am unteren Bein des Patienten.

8. Struktur nach Anspruch 2 oder 3, wobei der Verschluss wenigstens teilweise von der belastbar flexiblen Struktur getrennt werden kann.

9. Struktur nach Anspruch 8, wenn abhängig von Anspruch 2, wobei der genannte Verschluss erste und zweite Verschlussteile (82) umfasst, die starr an den entsprechenden der ersten und zweiten Kantenabschnitte (18, 20) befestigt sind sowie einen entfernbaren Verschlussgurt, der an beiden der genannten ersten und zweiten Verschlussteile (82) befestigt werden kann, um den zweiten schlauchförmigen Abschnitt (14) in der genannten geschlossenen Position zu sichern.

10. Struktur nach Anspruch 8, wenn abhängig von Anspruch 3, wobei der genannte Verschluss ein erstes Verschlussteil (82) umfasst, das starr am genannten zweiten schlauchförmigen Abschnitt (14) befestigt ist, ein zweites Verschlussteil (82), das starr am zweiten Kantenabschnitt (20) befestigt ist und ein entfernbarer Verschlussgurt, der an beiden der genannten ersten und zweiten Verschlussteile (82) befestigt werden kann, um den zweiten schlauchförmigen Abschnitt (14) in der genannten geschlossenen Position zu sichern.

11. Struktur nach Anspruch 3, wobei der genannte Verschluss ein erstes Verschlussteil (72) umfasst, das an einer externen Fläche des genannten ersten Kantenabschnitts (68) gesichert ist und ein zweites Verschlussteil (70), das an einer internen Fläche des genannten zweiten Kantenabschnitts (66) gesichert ist, wobei die ersten und zweiten Verschlussteile (70, 72) zusammen gesichert werden können, wenn der genannte zweite Kantenabschnitt (66) auf dem ersten Kantenabschnitt (68) aufliegt, um den genannten zweiten schlauchförmigen Abschnitt (64) in der genannten geschlossenen Position zu halten.

12. Struktur nach einem der vorstehenden Ansprüche, wobei die belastbar flexible Struktur (50) aus einem Silikonelastomer mit einer Shore-A-Härte von 30 bis 80 besteht.

13. Verfahren des Einpassens einer belastbar flexiblen Struktur (50) des Fußes und unteren Beins eines Patienten, um eine Fußgelenk-Fuß-Orthese (10; 60) zum Wiederstand einer Plantarflexion des Fußes des Patienten bereitzustellen, wobei das Verfahren Folgendes umfasst:
Bestimmen der Größe des Fußes des Patienten und eines Ausmaßes, in welchem der Patient unter der Fuß-Plantarflexion leidet;
Auswählen einer belastbar flexiblen Struktur (50) aus Silikonelastomer, deren Größe zum Einpassen eines Patienten mit einem Fuß der bestimmten Größe geeignet ist und dessen Belastbarkeit gegenüber der Plantarflexion für die Korrektur des bestimmten Ausmaßes geeignet ist, in welchem der Patient unter der Fuß-Plantarflexion leidet;
Einsetzen des Fußes des Patienten und wenigstens eines Abschnitts des unteren Beins des Patienten in die belastbar flexible Struktur (50);
Überlappen der ersten und zweiten Regionen (42, 44; 74, 76) eines ersten Abschnitts (12; 62) der belastbar flexiblen Struktur (50), in welche der Fuß des genannten Patienten eingesetzt worden ist, bis der genannte erste Abschnitt (12; 62) dicht um den Fuß des Patienten passt; und
das permanente Sichern der genannten ersten und zweiten Regionen (42; 44; 74; 76) miteinander.

14. Verfahren zur Herstellung einer belastbar flexiblen Struktur (50) zum Einpassen, gemäß dem in Anspruch 13 beanspruchten Verfahren, des Fußes und unteren Beins eines Patienten, um eine Fußgelenk-Fuß-Orthese (10; 60) zum Widerstand gegenüber einer Plantarflexion des Fußes des Patienten bereitzustellen, wobei das Verfahren Folgendes umfasst:
Herstellen eines Bestands eines belastbar flexiblen Silikonelastomers, das wenigstens nach dem Härten eine Belastbarkeit aufweist, die für den Widerstand des Ausmaßes einer Plantarflexion geeignet ist, die von einer bestimmten Patientenart erfahren wird;
Anwenden des belastbar flexiblen Silikonelastomers auf eine dreidimensionale Darstellung eines Fußes und eines unteren Beins für eine bestimmte Patientenart, um eine belastbar flexible Silikonelastomer-Struktur (50) zu bilden, die einen ersten Abschnitt (12; 62) und einen zweiten schlauchförmigen Abschnitt (14; 64) umfasst, der in einem Winkel zum ersten Abschnitt (12; 62) steht, wobei
der genannte erste Abschnitt (12; 62) derart konfiguriert ist, um erste und zweite Regionen (42; 44; 74; 76) zu enthalten, die miteinander kooperieren, um eine Öffnung zu definieren und in der Lage sind, überlappt zu werden und permanent aneinander gesichert werden, um einen Schlauch zu bilden, und
der genannte zweite schlauchförmige Abschnitt (14; 64) in der Lage ist, für das Einsetzen des Fußes eines Patienten in den durch den genannten ersten Abschnitt (12; 62) gebildeten Schlauch und für das Einsetzen wenigstens eines Teils des unteren Beins des Patienten in den genannten zweiten Abschnitt (14; 64) geöffnet zu werden;
Befestigen eines Verschlusses (34; 66, 68; 82; 84, 86) an den genannten zweiten Abschnitt (14; 64), wobei der genannte Verschluss (34; 66, 68; 82; 84, 86) derart betrieben wird, um am genannten zweiten Abschnitt (14; 64) in einer geschlossenen Position gesichert zu werden, in welcher der zweite schlauchförmige Abschnitt (14; 64) dicht um das untere Bein eines Patienten passt; und
Aushärten des belastbar flexiblen Silikonelastomers.

15. Fußgelenk-Fuß-Orthese (10; 60), die aus der belastbar flexiblen Struktur (50) nach einem der Ansprüche 1 bis 12 gebildet ist, wobei: sich die genannten ersten und zweiten Regionen (42, 44; 74, 76) des genannten ersten Abschnitts (12; 62) überlappen und permanent aneinander gesichert sind, um einen Schlauch zu bilden, der dicht um den Fuß des Patienten passt.

## Revendications

1. Structure élastiquement souple (50) devant être formée en une orthèse cheville-pied (10 ; 60) qui est configurée pour résister à une flexion plantaire du pied d'un patient, la structure élastiquement souple (50) comprenant une première partie (12 ; 62) et une seconde partie (14 ; 64) située à un angle par rapport à ladite première partie (12 ; 62), dans laquelle :
ladite première partie (12 ; 62) comprend des première et seconde régions (42, 44 ; 74, 76) qui coopèrent pour définir une ouverture et sont configurées et agencées de façon à être aptes à se chevaucher et aptes à être fixées de façon permanente l'une à l'autre pour former un tube qui s'ajuste étroitement autour du pied du patient ;
ladite seconde partie (14 ; 64) est formée sous la forme d'un tube fendu qui est apte à être ouvert pour l'introduction du pied du patient dans le tube formé par ladite première partie (12 ; 62) et pour l'introduction d'au moins une partie de la jambe inférieure du patient dans ladite seconde partie (14 ; 64), et
ladite structure élastiquement souple (50) comprend en outre une fermeture (34 ; 70, 72 ; 82 ; 84, 86) pour fixer ladite seconde partie (14 ; 64) dans une position fermée dans laquelle en utilisation la seconde partie (14 ; 64) s'ajuste étroitement autour de la jambe inférieure du patient ; la structure élastiquement souple (50) est faite d'élastomère de silicone et a une résilience qui est choisie pour être suffisante pour fournir une résistance à la flexion qui est appropriée pour la correction du degré particulier de flexion plantaire subie par le patient.

2. Structure selon la revendication 1, dans laquelle la seconde partie tubulaire (14, 64) est fendue longitudinalement pour définir une fente d'introduction, des première et seconde parties de bord de la fente étant configurées pour être sensiblement adjacentes l'une à l'autre lorsque la seconde partie tubulaire (14 ; 64) est dans la position fermée.

3. Structure selon la revendication 1, dans laquelle la seconde partie tubulaire (14, 64) comprend des première et seconde parties de bord (18, 20, 30 ; 66, 68), ladite seconde partie de bord (20, 30 ; 66) étant séparable de la première partie pour permettre à la seconde partie tubulaire (14 ; 64) d'être ouverte et étant configurée pour recouvrir la première partie de bord (18 ; 68) lorsque la seconde partie tubulaire (14 ; 64) est dans la position fermée.

4. Structure selon l'une des revendications 2 ou 3, dans laquelle la fermeture (34 ; 70 ; 72 ; 82 ; 84, 86) est attachée de façon fixe à la structure orthétique.

5. Structure selon la revendication 4, dans laquelle la fermeture comprend une première partie (84) attachée de façon fixe à ladite seconde partie tubulaire (14) et une seconde partie (86) attachée de façon fixe à la seconde partie de bord (30), au moins une extrémité libre de la seconde partie (86) étant apte à être fixée à la première partie (84) pour fixer la seconde partie tubulaire (14) dans ladite position fermée.

6. Structure selon la revendication 5, dans laquelle la première partie et au moins une partie de ladite seconde partie comprennent des parties complémentaires appropriées d'un système de fixation mécanique à boucles et crochets pour permettre à la seconde partie d'être fixée à ladite première partie.

7. Structure selon la revendication 4 prise en dépendance de la revendication 3, dans laquelle la fermeture comprend une sangle (34) attachée de façon fixe à une extrémité à ladite seconde partie de bord (30) au moyen d'une fixation (36), ladite sangle (34) étant configurée pour être passée autour de toute la circonférence de la seconde partie tubulaire (14) avant d'être fixée à elle-même pour solliciter la seconde partie tubulaire (14) vers la position fermée, l'agencement étant tel que la fixation (36) prend appui sur ladite première partie de bord (18) lorsque la seconde partie tubulaire (14) est dans la position fermée et non sur la jambe inférieure du patient.

8. Structure selon l'une des revendications 2 ou 3, dans laquelle la fermeture est au moins partiellement séparable de la structure élastiquement souple.

9. Structure selon la revendication 8 prise en dépendance de la revendication 2, dans laquelle ladite fermeture comprend des première et seconde parties de fermeture (82) attachées de façon fixe à des parties respectives desdites première et seconde parties de bord (18, 20), et une bande de fermeture amovible apte à être attachée à la fois à ladite première et à ladite seconde partie de fermeture (82) pour fixer la seconde section tubulaire (14) dans ladite position fermée.

10. Structure selon la revendication 8 prise en dépendance de la revendication 3, dans laquelle ladite fermeture comprend une première partie de fermeture (82) attachée de façon fixe à ladite seconde section tubulaire (14), une seconde partie de fermeture (82) attachée de façon fixe à la seconde partie de bord (20), et une bande de fermeture amovible apte à être attachée à la fois à ladite première et à ladite seconde partie de fermeture (82) pour fixer la seconde section tubulaire (14) dans ladite position fermée.

11. Structure selon la revendication 3, dans laquelle ladite fermeture comprend une première partie de fermeture (72) fixée à une surface externe de ladite première partie de bord (68) et une seconde partie de fermeture (70) fixée à une surface interne de ladite seconde partie de bord (66), lesdites première et seconde parties de fermeture (70, 72) étant aptes à être fixées ensemble lorsque ladite seconde partie de bord (66) recouvre la première partie de bord (68) pour retenir ladite seconde partie tubulaire (64) dans ladite position fermée.

12. Structure selon l'une quelconque des revendications précédentes, dans laquelle la structure élastiquement souple (50) est faite d'un élastomère de silicone ayant une dureté au duromètre Shore A de 30 à 80.

13. Procédé d'ajustement d'une structure élastiquement souple (50) au pied et à la jambe inférieure d'un patient pour fournir une orthèse cheville-pied (10 ; 60) pour résister à une flexion plantaire du pied du patient, le procédé comprenant :
déterminer la taille du pied du patient et d'un degré auquel le patient souffre d'une flexion plantaire du pied ;
sélectionner une structure élastiquement souple (50) d'élastomère de silicone dont la taille est appropriée pour s'ajuster à un patient ayant un pied de la taille déterminée et dont la résilience à une flexion plantaire est appropriée pour la correction du degré déterminé auquel le patient souffre d'une flexion plantaire du pied ;
introduire le pied du patient et d'au moins une partie de la jambe inférieure du patient dans la structure élastiquement souple (50) ;
chevaucher les première et seconde régions (42, 44 ; 74, 76) d'une première partie (12 ; 62) de la structure élastiquement souple (50) dans laquelle le pied du patient a été introduit jusqu'à ce que ladite première partie (12 ; 62) s'ajuste étroitement autour du pied du patient ; et
fixer de façon permanente lesdites première et seconde régions (42 ; 44 ; 74 ; 76) ensemble.

14. Procédé de fabrication d'une structure élastiquement souple (50) destinée à s'ajuster, conformément au procédé selon la revendication 13, au pied et à la jambe inférieure d'un patient pour fournir une orthèse cheville-pied (10 ; 60) pour résister à une flexion plantaire du pied du patient, le procédé comprenant :
préparer une quantité d'élastomère de silicone élastiquement souple qui a une résilience, au moins lorsqu'il est durci, qui est appropriée pour résister au degré de flexion plantaire subie par un type de patient particulier ;
appliquer l'élastomère de silicone élastiquement souple à une représentation tridimensionnelle d'un pied et d'une jambe inférieure pour un type de patient particulier pour former une structure en élastomère de silicone élastiquement souple (50) qui comprend une première partie (12 ; 62) et une seconde partie tubulaire (14 ; 64) située à un angle par rapport à ladite première partie (12 ; 62), dans laquelle
ladite première partie (12 ; 62) est configurée pour comprendre des première et seconde régions (42 ; 44 ; 74 ; 76) qui coopèrent pour définir une ouverture et sont aptes à se chevaucher et à être fixées de façon permanente l'une à l'autre pour former un tube, et
ladite seconde partie tubulaire (14 ; 64) est apte à être ouverte pour l'introduction d'un pied d'un patient dans le tube formé par ladite première partie (12 ; 62) et pour l'introduction d'au moins une partie de la jambe inférieure du patient dans ladite seconde partie (14 ; 64) ;
attacher une fermeture (34 ; 66, 68 ; 82 ; 84, 86) à ladite seconde partie (14 ; 64), ladite fermeture (34 ; 66, 68 ; 82 ; 84, 86) étant actionnable pour fixer ladite seconde partie (14 ; 64) dans une position fermée dans laquelle la seconde partie tubulaire (14 ; 64) s'ajuste étroitement autour de la jambe inférieure d'un patient ; et
laisser durcir l'élastomère de silicone élastiquement souple.

15. Orthèse cheville-pied (10 ; 60) formée à partir de la structure élastiquement souple (50) de l'une des revendications 1 à 12, dans laquelle : lesdites première et seconde régions (42, 44 ; 74, 76) de ladite première partie (12 ; 62) se chevauchent et sont fixées de façon permanente l'une à l'autre pour former un tube qui s'ajuste étroitement autour du pied du patient.
